# EUROPEAN PATENT APPLICATION

(11) **EP 1 426 766 A1**
(43) Date of publication of application: **09.06.2004**
(21) Application number: 02026846.2
(22) Date of filing: 29.11.2002
(51) Int. Cl.: G01N 33/68

(54) **Differential diagnosis of breast cancer by protein profiling using mass spectrometry**

(71) Applicant: König, Christine, 22529 Hamburg (DE); Fuhrmann, Gesa Katharina, 22880 Wedel (DE); von Bergen, Martin, 21483 Gulzow (DE)
(72) Inventor: König, Christine, 22529 Hamburg (DE); Fuhrmann, Gesa Katharina, 22880 Wedel (DE); von Bergen, Martin, 21483 Gulzow (DE)
(74) Representative: UEXKÜLL & STOLBERG

(57) **Abstract**

The present invention relates to methods for generating a data base for differential diagnosis of breast cancer stages, which method comprises steps, wherein:
(a) samples are obtained from healthy subjects, from subjects having a ductal carcinoma in situ (DCIS) and from subjects with invasive, ductal breast carcinoma;
(b) protein profiles are analyzed in all samples using mass spectrometry and entered into a data base;
(c) a mathematical algorithm is used to characterize the protein profiles analyzed as specific for healthy subjects, specific for subjects having a ductal carcinoma in situ or specific for subjects having invasive, ductal breast cancer.

## Description

The present invention relates to methods for generating a data base for differential diagnosis of breast cancer stages, which method comprises steps, wherein:
(a) samples are obtained from healthy subjects, from subjects having a ductal carcinoma in situ (DCIS) and from subjects with invasive, ductal breast carcinoma;
(b) protein profiles are analyzed in all samples using mass spectrometry and entered into a data base;
(c) a mathematical algorithm is used to characterize the protein profiles analyzed as specific for healthy subjects, specific for subjects having a ductal carcinoma in situ or specific for subjects having invasive, ductal breast cancer.

The differential diagnosis of breast cancer stages uses the above data base and further comprises steps, wherein
(d) a sample is obtained from a subject;
(e) the protein profile of the sample is analyzed and compared to the data base of (b); and
(f) on the basis of the characteristics of the protein profile the subject is diagnosed as healthy, as having a ductal carcinoma in situ or as having invasive, ductal breast cancer.

Breast cancer or mammary carcinoma is a severe disease and with an incidence of about 10% in women one of the major health problems of today. The vast majority (over 80%) of the carcinomas are ductal carcinomas originating from terminal ductulo-lobular units (TDLUs).

However, the cancer is diagnosed in 80% of the cases only at a point at which the tumor has already grown into surrounding tissue of the breast and is an invasive tumor. Only in 20% of the cases the tumor can be detected when still growing intraductal. For women having a pre-disposition for breast cancer (due to transmission from the family or due to a diagnosed genetic defect) and women without such a pre-disposition only few non-invasive diagnostic methods are available for analyzing the state of the breast.

Early detection of breast cancer can result from palpation. Women who are aware of the necessity of a respective analysis can perform this form of early diagnosis by themselves. To ensure early tumor detection it is, however, decisive that the analysis is performed regularly and with high quality. From the 30th year onwards a respective analysis for early detection of tumors should be performed by a gynaecologist as a part of the cancer prevention. If no specific evidence for a breast cancer or a pre-disposition of the patient is present, the analysis should be performed by palpation.

Palpation is rather unsafe, as the tissue structure of the breast is strongly influenced by the cycle phase, the size of the organ, the age of the patient and other factors. Further, the experience of the clinician has a significant influence in the diagnosis. A differentiated macroscopic visualization of the tissue structure of the organ or of the internal regions can be performed via mammography or sonographic analysis. Again, the quality of the diagnosis is dependent on the above-identified parameters, specifically the method is highly dependent on the experience and qualification of the clinician. Incorrect diagnosis is often based on the fact that non-malignant diseases of the breast lead to tissue modifications which cannot be distinguished from malignant diseases via mammography.

The known diagnostic methods based on protein detection are presently applied after diagnosis of breast cancer using mammography and not for detection of breast cancer in a screening method. Diagnostic methods used in this method for identification of different tumor stages can be distinguished as follows:
(1) ELISA (Enzyme-Linked-Immuno-Sorbent-Assay)
   The ELISA is the oldest and most frequently used method for detection of single proteins in homogenates of body fluids. The best known example for detection of tumor markers is the detection of the prostate specific antigen (PSA), which is used for diagnosis and control of developing prostate cancer. A requirement for detecting proteins via antibodies in general is the availability of suitable antibodies, which are sufficiently specific and sensitive to allow a precise diagnosis. Until today there is no ELISA based diagnosis method for breast cancer. Efforts to use the tumor marker CA15-3 for the early detection of breast cancer failed. The sensitivity of the test was only 23% and the specificity only 69% (Stieber et al., 2001). CA15-3 is, however, still used for analysing the progression of the therapy in advanced breast cancer
(2) Immunohistochemical methods
   The use of immunohistochemical methods for identification of tumor marker molecules is only applicable for more detailed analysis of progressed or extended tumors, since for this purpose a tissue probe has to be obtained from the patient. In the case of a breast cancer detection of hormone receptors (e.g. estrogen receptor) and growth factor receptors (e.g. c-erb-b2/HER2) is for example used to estimate the chances of a therapy directed towards the respective receptor proteins (i.e. a therapy using Tamoxifen or Herceptin). Detection of increased concentrations of tumor suppressor protein p53 or one of its mutated and metabolically stabilized forms via immunohistochemical analysis also serves to evaluate the chances of a therapy based on a programmed cell death, so-called apoptosis (for example via radiation theraPY).
(3) Surface Enhanced Laser Desorption Ionization-Mass Spectrometry (SELDI-MS):
   The SELDI-MS technology is used for diagnostic methods in two alternative approaches. According to the first approach only one specific marker (protein) is analyzed. The analysis method comprises for example the coupling of an antibody to a carrier for probes and specific binding of the protein from the mixture (see Aß-kit of Ciphergen; Austen et al., 2000) .
   According to the alternative approach a protein profile is obtained and the entire spectrum or a part thereof is used for a complex analysis (Ardekani et al., 2002; Ball et al., 2002; Petricoin et al., 2002). In the publication of Petricoin et al. for example sera from patients with ovarian cancer were analyzed using SELDI. A reliability of 94% for distinguishing between healthy subjects and cancer patients shows that power of the method.
   In a recent paper diagnosis of breast cancer using serum probes and SELDI analysis is discussed (Li et al., 2002). In this analysis the progression stage of the cancer was classified using the guidelines of the UICC. Accordingly, DCIS (T0) and 38 invasive tumors smaller than 2cm (T1) were not further distinguished and included into a single group (T0 + T1). This publication thus cannot enable diagnosis of the predominating form of intraductal carcinomas (i.e. DCIS) but merely distinguishes late cancer stages amongst each other. Of course, it is much easier to find markers for later cancer stages, specifically for breast cancer stages, wherein the cancer has invaded the tissue surrounding the ductus, in which the cancerous cells are likely to secrete metabolites into the blood.

A method for early and safe detection of breast cancer at the intraductal DCIS stage would have significant advantages. The main advantage of detecting breast cancer at the DCIS stage resides in the fact that if margin width of resected cancerous tissue is big enough after surgery, the chances for complete healing (i.e. survival rate of over 95% 5 years after treatment, Silverstein et al., 1995) after resection of the cancerous tissue are much better than if the respective treatment is carried out after diagnosis of cancer at any of the stages TI to TIV. A method that would allow early detection of DCIS in a safe but simple diagnostic screening test would thus significantly improve the healing rate.

Further, due to the difficulties in classifying a tumor as DCIS according to the methods presently available many patients are possibly over treated. A number of patients still receive mastectomy or very extensive resection due to the better prognosis, although this would not necessarily be required at that stage or would not be required at all. The clinical outcome of DCIS in the individual patient is unknown and until today the only widely accepted prognostic factor is the tumor free margin width of the removed tissue material. Mastectomy, although offering cure rates of more than 95% (Silverstein et al., 1995a; Solin et al., 1996; Talamonti, 1996), is considered to be an over treatment in a number of cases, specifically for DCIS patients. In consideration of the severe medical and psychological problems associated with mastectomy, it would be highly desirable to have a method for safe diagnosis of the DCIS stage of breast cancer.

Chemo- and radio-therapy does not significantly improve healing chances during DCIS stage but only during later stages of breast cancer (Petera et al., 2001). Unambiguous detection of DCIS could thus reduce the treatment regimen, i.e. avoid chemo- and/or radio-therapy.

A diagnosis method for DCIS would also allow to screen patients with DCIS for tumor development using a tight screening regimen, such as regular mammography in short intervals. The diagnosis method would also allow to assess the success of preventive medication.

The present invention addresses these problems.

In accordance with the present invention a method for generating a data base for differential diagnosis of breast cancer stages is provided, which method comprises steps, wherein:
(a) samples are obtained from healthy subjects, from subjects having a ductal carcinoma in situ (DCIS) and from subjects with invasive, ductal breast carcinoma;
(b) protein profiles are analyzed in all samples using mass spectrometry and entered into a data base;
(c) a mathematical algorithm is used to characterize the protein profiles analyzed as specific for healthy subjects, specific for subjects having a ductal carcinoma in situ or specific for subjects having invasive, ductal breast cancer.

The data base can subsequently be used in an in vitro method for differential diagnosis of breast cancer stages, comprising steps, wherein
(d) a sample is obtained from a subject at risk of having breast cancer;
(e) the protein profile of the sample is analyzed and compared to the data base of (b); and
(f) on the basis of the characteristics of the protein profile the subject is diagnosed as healthy, as having a ductal carcinoma in situ or as having invasive, ductal breast cancer.

The present inventors have surprisingly found that even in the intraductal DCIS stage of breast cancer tumor markers can be found in serum and can be characterized as specific for DCIS or as specific for a subsequent breast cancer stage by analysis of the protein profiles, specifically using mass spectrometry. The method of the present invention is easy to carry out and provides little room for human error. Computer based data analysis excludes subjective analysis of biological material by humans (such as in analyses of immunohistochemical staining of tumor material). Experimental variation of the method is low when compared to other methods for analysis of biological material, which require high expertise of the user (for example RNAse-free tissue processing, mRNA extraction, quantiative RT-PCR etc.).

The differential diagnosis of breast cancer stages of the present invention can easily be developed for serial screening and should be applied as a standard for women from 30 years onwards. Easy processing and high safety makes this method an attractive method for yearly cancer prevention diagnosis of women.

For the analysis of protein profiles a mouse model was developed which reproduces the course of the disease (Schulze-Garg et al., 2000; DE 199 03 371). This mouse model was now used for exemplifying the method for preparing a data base and the method for diagnosing early breast cancer stages according to the present invention.

In accordance with the present invention the term "ductal carcinoma in situ" is used to refer to a breast cancer stage, wherein the tumor is present exclusively in the ductus. This stage can thus be distinguished from any other ductal breast cancer that has infiltrated or micro-invaded the surrounding tissue. For this purpose, mammary glands are isolated embedded into parafin and a number of sections are histologically analyzed using the pathological criteria outlined in more detail below. According to the tissue it originated from it can be distinguished between carcinoma ductale in situ (DCIS or CDIS) and carcinoma lobulare in situ (CLIS). The latter form is a rare event and in the present application both forms are referred to as DCIS.

Further, the term "protein profile" is used to refer to features such as size and ionic strength of individual proteins in a mixture of proteins. The protein mixture can thus be characterized by analysis of specific features such as size and amount of individual proteins of the mixture. The size can further reflect characteristic post-translational modifications of proteins, such as phosphorylation, glycosylation, acetylation etc.

In accordance with the present invention, the protein profiles are preferably analyzed via mass spectrometry, specifically surface enhanced laser desorption ionisation mass spectrometry (SELDI-MS). The mass range selected for further analysis is preferably the range of 5 to 200 kDa.

The mathematical algorithm used to characterize the protein profiles analyzed is any algorithm which allows to discriminate the different protein profiles of the tumor stages of interest with high consistency. Preferably a competitive learning and model selection approach is used. For example, several artificial neuronal net models can be designed to discriminate in parallel between protein profiles from different tumor stages of interest and those obtained from healthy subjects. For this purpose, protein profiles are processed, single parameters are statistically evaluated and a mutual information test is performed on the same. The parameters are ranked according to their informational content and the most promising combinations of parameters (e.g. peaks, masses, classificators) are selected for the generation of artificial neuronal net models.

These pre-trained artificial neuronal net models can further be optimized and trained using a training data set. This data set preferably consists of protein profiles from specimen pre-selected on the basis of a gold standard diagnosis. The selected artificial neuronal net can further be validated by applying a test data set and cross evaluating training and test data set. Finally, the artificial neuronal net, which provided the best results in the cross evaluation test can be selected and an independent testing data set can be applied.

The present invention further provides a method of identifying one or more proteins specific for subjects having ductal carcinoma in situ, which method comprises the use of a data base according to the present invention and further comprises steps, wherein one or more proteins characterized as specific for ductal carcinoma in situ are isolated. In compliance with one aspect of the present invention the proteins identified in the initial analysis can be isolated subsequently and used in isolation for the diagnosis method. A protein is identified as specific for DCIS breast cancer, if the protein has any feature that makes it identifiable in the protein profiling step. This feature may but not be a specific amino acid sequence, charge, mass or size it can further reside in the characteristic relative concentration of the protein in a sample. In accordance with this embodiment of the methods of the present invention, preferably the sequence of the isolated protein is further obtained.

In a further aspect of the present invention an in vitro method for differential diagnosis of breast cancer stages, is provided, which comprises the above method of identifying one or more proteins specific for ductal carcinoma in situ and further comprises steps, wherein a probe is obtained from a subject at risk of having breast cancer and analyzed for the presence of the protein(s) specific for ductal carcinoma in situ, wherein the subject is diagnosed as having ductal carcinoma in situ if the protein(s) is (are) present in the probe. In other words, once the proteins specific for DCIS are identified, the diagnosis need not rest on the analysis of the protein profiles of the entire protein mixture in a sample but may well focus on the specific proteins.

A Protein identified as a peak or a mass in the SELDI analysis as a protein with specificity for DCIS, can further be identified, isolated and analyzed by a number of alternative methods well known in the art such as:
1. Using an interface the same probe on a chip as used for SELDI-MS analysis may be applied to a conventional MALDI-MS-MS. In this analysis the peak of interest from a first MS can be selectively analyzed in a second MS. The mother ion from the first MS step will hereby dissociate into daughter ions. The sequence of the mother ion can directly be deduced from the masses of the daughter ions.
2. A 2D-gelelectrophoresis can alternatively be performed. Since the isoelectric point (pI) and mass of the protein are known from the SELDI-MS data, the identification of the corresponding spot (protein) in a 2D-gel is possible. Purification of the protein from the gel and subsequent tryptic digestion according to methods well known in the art will yield a mixture of peptide fragments. MALDI-TOF analysis of such tryptic fragments will provide characteristic fingerprints upon which will allow identification of known proteins using sequence data bases.
3. After enrichment of the protein of interest via ion exchange chromatography or size exclusion chromatography a further alternative approach may use electro spray ionization-MS (ESI-MS). ESI-MS of the enriched protein in row with a second MS can also yield the sequence of the protein of interest. In analogy to the method described under 1., above, a further MS analysis of the mother ion after fast atomic bombardment which will yield daughter ions from whose masses the sequence of the mother ion can be deduced.

The identification of known proteins via the methods described above may further be confirmed with conventional immuno-affinity-techniques (e.g. western-blotting, ELISA, immunocyto-immuno-histochemistry a.o.).

According to this embodiment a protein specific for subjects having ductal carcinoma in situ can be prepared, in a method comprising the above identification of a protein specific for ductal carcinoma in situ and recombinant or synthetic preparation of the protein identified. Methods of recombinant or synthetic preparation of proteins are well known in the art and this way of proceeding enables obtaining of proteins specific for ductal carcinoma in situ.

According to the present invention the methods are preferably used for diagnosis of breast cancer in humans. Consequently, according to a preferred embodiment, the subjects used for generating the data base are humans.

Specific advantages may be obtained in an alternative embodiment, wherein the subjects used for generating the data base are mice or transgenic mice diagnosed to have a ductal carcinoma in situ. Using animals for generation of the data base allows the analysis of a large population of subjects which all have the same confirmed stage of cancer.

The samples obtained in steps (a) and (d) can be any sample which provides a protein profile with characteristic features for DCIS. Preferably the samples are blood samples, serum samples and/or biopsy samples.

One of the major advantages of the present invention resides in the fact that the diagnosis method can be performed using blood samples or serum samples, since the same can easily be obtained and processed.

According to a preferred embodiment, the samples obtained in step (d) of the above method are serum samples which are purified by depletion of hemoglobin prior to mass spectrometry. The proteins are then loaded or coated onto the surface used for SELDI-MS analysis.

Further advantages are obtained if the method of the present invention is carried out using surfaces which are weak cation exchange chips or strong anion exchange chips.

### Brief Description of the Figures

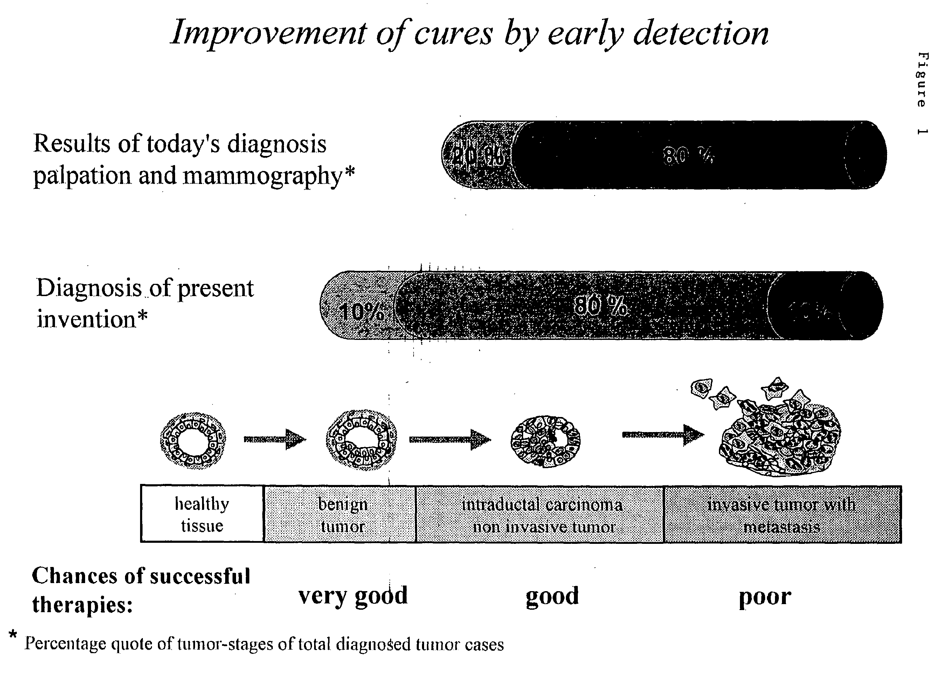

- Fig. 1:: Shows how the diagnosis method of the present invention significantly improves cure rates.

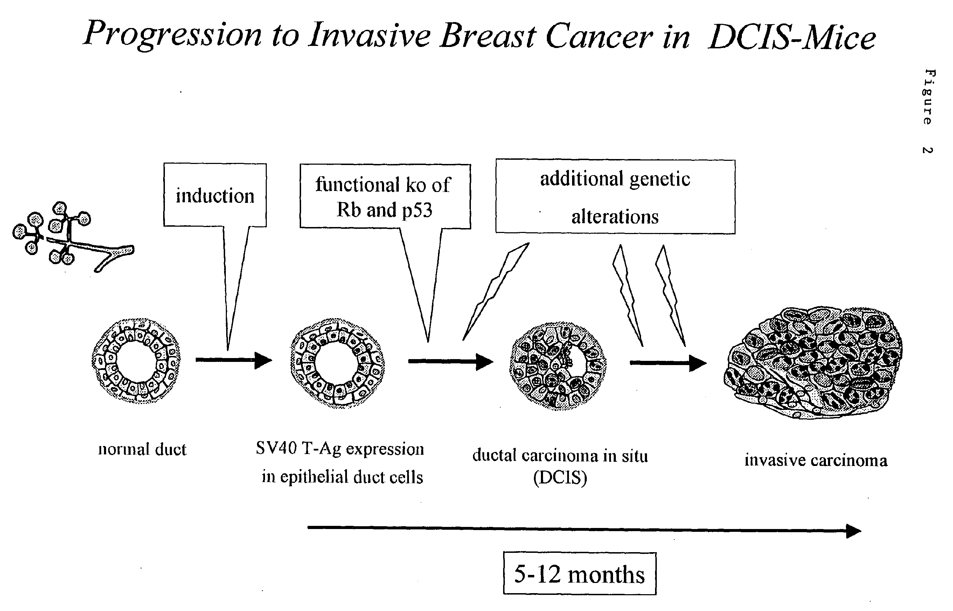

- Fig. 2:: Shows the different breast cancer stages and thus the development of the cancer from DCIS to invasive carcinoma.

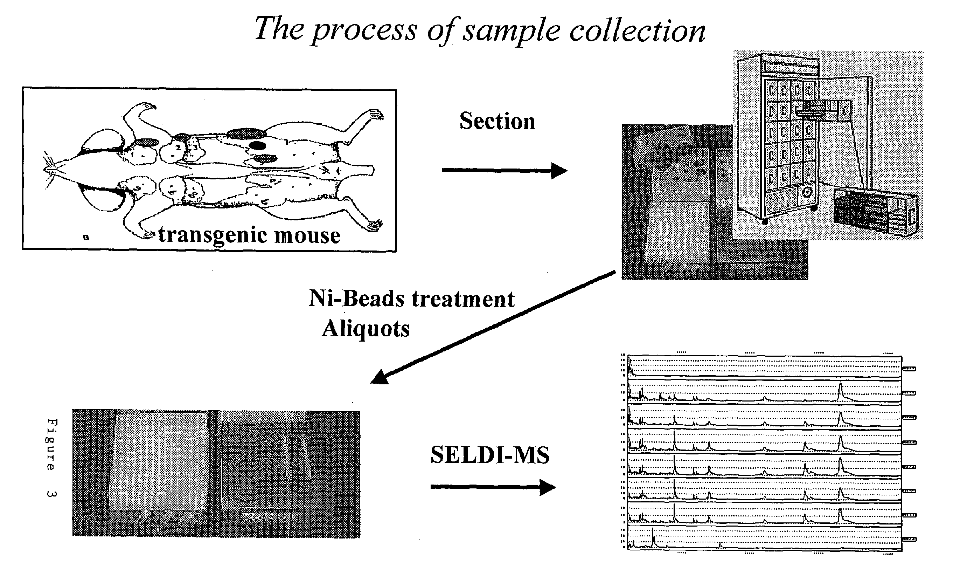

- Fig. 3:: Summarizes the process of sample collection and analysis as described in the following examples.

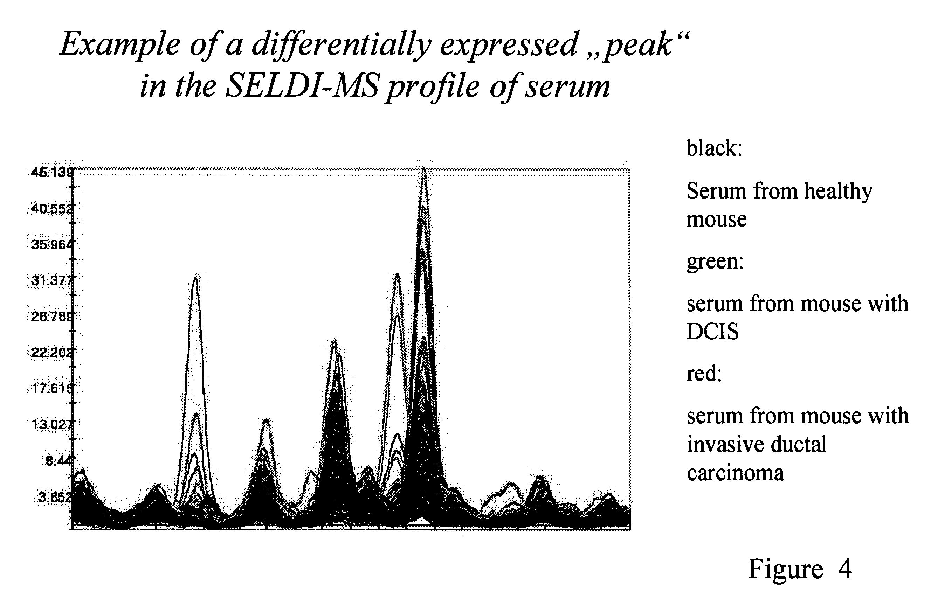

- Fig. 4:: Provides an Example of a differentially expressed peak in the SELDI-MS profile of serum probes analyzed in accordance with the present invention.

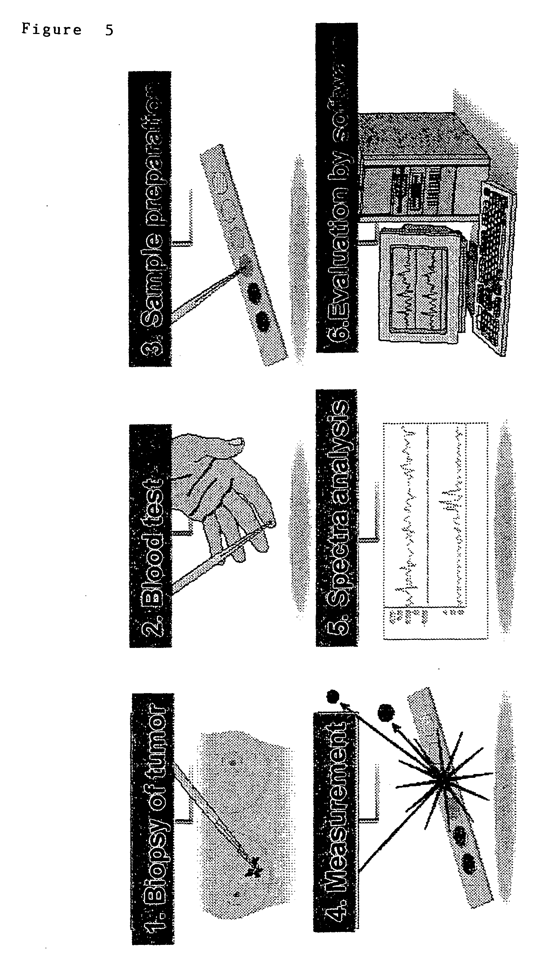

- Fig. 5:: Summarizes the diagnostic method according to the present invention. It should be noted that the Figure provides for analysis of biopsy and blood samples, whereas it can be sufficient to analyze one of the samples (biopsy or blood sample).

### EXAMPLES

In the following examples protein profiles from serum and biopsies of transgenic mice was used to determine whether the DCIS stage of breast cancer can be identified with this method.

### Animals

Mice carrying different transgenic gene sequences which will develop tumor at a predictable age have been described in the prior art (Schulze-Garg, 2000). The animals differ in the incidence of tumor generation and the time of latency. Animals of the line WAP-T-NP8 form tumors with a high prevalence of 83% after 5 +/- 1,5 months.

For the protein profiling analysis of protein profiles mice were selected using very strict pathological selection criteria. Only animals, having an unambiguous histopathological finding, which allowed to assign the cancer in the animal to a certain progression state were used. Further, only those progression states were used for serum screening, for which at least five animals per stage were available.

**Table 1**

| Data set from transgenic animals used for serum screening. | | | |
|---|---|---|---|
| Transgenic Mouse Strain | Normal | DCIS | Cancer |
| WAP-T-NP8 | 20 | 32 | 32 |

### Pathological analysis of probes from the mice

For assessing atypical tissue in general only parameters such as size and heterogeneity of nuclei, shape, form, chromatin structure, nuclei and the number of mitoses are assessed beside the size of the cells (Boecker et al., 1997). For diagnosis of DCIS in the present case at least three of the following criteria should be met in cases where no characteristic morphology, such as clinging type or cribriform DCIS was observed (Cardiff, 2001) :
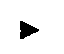 basophilic cells;
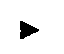 several rows of epithelium;
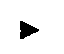 number of mitoses;
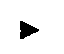 number of apoptosis; and
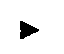 polymorphism and anaplasia of the nuclei.

### Obtaining Probes

For retro-orbital blood sampling the animals were anaesthetized with chloroform and subsequently sacrificed according to a standard protocol. Organs and probes were obtained for further analysis.

### Obtaining and Processing Serum

Blood obtained as outlined above is maintained over night at 4°C until spontaneous blood clotting and contraction of the blood cakes can be observed. After centrifugation for 10 minutes at 10,000 x g at 4°C the serum is transferred cell-free into a clean Eppendorf tube, split up into aliquots and further processed according to the following protocol.

Serum aliquots are purified by treatment with "Nickel-beads" (subsequently designated Ni-beads) from potentially present hemoglobin derived from lysed erythrocytes, and stored until further use at -20°C. Serum aliquots which were not directly used are shockfrozen at -80°C and stored just as the tissue probes.

To reduce free hemoglobin 51 µl of the original serum is centrifuged (15 min., 4°C, 13000 rpm). At the same time the Ni-beads (Ni-CAM HC Resign High Capacity Nickel Chelate Affinity Matrix, Sigma) are mixed with an equal volume of 1 xPBS/0,3 M NaCl and subsequent centrifugation (2 min, 4,5 x g, room temperature) and washed. Washing is repeated three times.

Subsequently, 50 µl of the centrifuged serum is mixed with an equal volume of Ni-beads pretreated as outlined above in a fresh Eppendorf tube and incubated for 20 minutes at 4°C with shaking (1000 UPM). The Ni-beads are pelleted via centrifugation (2 Min, 4,5 x g, 4°C) and the supernatant is transferred into a fresh Eppendorf tube. The hemoglobin depleted serum is split up into aliquot portions of 2 µl and frozen at -20°C.

### Application of Probes and Mass Spectrometric Analysis

For analysis via SELDI-MS various types of chips were tested. In these tests it was found that chips with weak cationic or strong anionic exchanger properties are superior (e.g. WCX-2 or SAX-2 chip, "Weak Cation Exchange"). The chips were chosen on the basis of the results obtained using different binding conditions (ionic strength, pH, detergent). The results were compared with respect to number of and reproducibility of peaks in the mass range of interest (5-200 kDa) and chips providing the best results were selected.

The "Bioprocessor" (Ciphergen, Paolo Alto, USA) can process 23 probes in parallel. Each probe is applied per chip in four replicas. Further, a buffer control and a mixture of known marker proteins in known concentrations is also included in four replicas for internal calibration of the analysis. All steps are carried out at room temperature.

For applying the probes to the chip surface an aliquot of hemoglobin depleted serum probes is thawed on ice and diluted in a ratio of 1/20 in binding buffer (50 mM NH4-acetate, pH 4.5,4 0.1% Triton X-100). 2 µl of the aliquot probe are mixed with 38 µl binding buffer, briefly centrifuged at 16.1 x g and incubated at room temperature for at least 30 minutes. Directly prior to applying the probe onto the chip, the probe is briefly mixed.

The surfaces of the chip to be inserted into the bioprocessor are activated by adding 2 x 100 µl 10 mM HCl for 5 minutes on a shaker at 200 rpm. Subsequently they are washed three times with 100 µl H₂0 and 2 x 100 µl binding buffer, each for 5 minutes with shaking at 200 rpm. After these preparatory steps, 5 µl of the prepared serum probes or control probes are pipetted into a 100 µl binding buffer (50 mM NH₄-acetate, pH 4.5, 0.1% Triton X-100). For each spot a new pipette tip is used. The entire bioprocessor is sealed with foil and incubated for 45 minutes with shaking at 200 rpm. Subsequently the chip is washed 2 x 5 minutes and 3 x 2 minutes with 100 µl binding buffer, as well as 1 x 5 minutes with 100 µl H₂O with shaking at 200 rpm. The chips are taken out of the bioprocessor and placed in a right angle on paper to remove residual fluid. The chip is dried for 40 minutes at 37°C. To the dried chip 2 x 0.6 µl freshly prepared matrix (SPA in 50% aceto nitrile, 0.5% TFA) is added per spot using a fresh pipette tip for each spot. The chip is then analyzed using a standard scheme for detection in mass spectrometry:

Instrument: PBS-II high mass: 200,000 Da, digitizer rate: 250.0 MHz, ion mode: positive chamber vacuum: 5.980 e - 0.07 Torr, source voltage: 20,000 V, detector voltage: 1,800 V, time lag focusing: on, pulse voltage: 3,000 V, pulse lag time: 1,601 ns, focus mass: 50,000.00 Da, shots fired: 273, shots kept: 247, high: 0, low: 0, intensity low: 235, intensity high: 290, sensitivity low: 8, sensitivity high: 8.

The resulting spectra were exported as csv data files and read into a classification system for assessing the data.

### Results

Protein profiles were obtained by binding the proteins to different chromatographic surfaces obtainable from Ciphergen. Various parameters such as buffer salt, pH, total salt content, presence in concentration of detergents, etc. were analyzed. The surfaces selected were strong anion exchange surfaces (SAX2-chip) and a weak cation exchange surface (WCX2-chip).

Spectra in a mass range of 5-200 kDa were obtained which covers small peptide molecules as well as bigger proteins. The choice of the range starting at 5 kDa was necessary, since below 5kDa the background noise of the almost saturated detector is too high to allow reproducible analysis. Above 200 kDa there are hardly any reproducible peaks due to the reduced ion character in proteins of high masses.

Noise reduction according to the software of the producer was used in general. Results were obtained by identifying peaks with the software delivered with the apparatus. Only peaks with an intensity of at least 3 times of that obtained as background noise were used for the analysis.

The selection of the statistical relevant peaks was performed using the Ciphergen software package delivered with the instrument. First, the peaks from the spectra were identified and then the peaks were compared the software Comparison Wizard.

The peaks were subsequently applied to an artificial neuronal net to identify those peaks which allow to distinguish between spectra of different stages. The 17 masses listed in table 2 were identified as markers with differential character for DCIS or invasive breast cancer stages using the WCX-2 chip.

**Table 2:**

| **Table 2: Masses of markers of the DCIS mouse model** | |
|---|---|
| Marker | m/z |
| BCM01 | 5220 |
| BCM02 | 5240 |
| BCM03 | 5579 |
| BCM04 | 5590 |
| BCM05 | 5630 |
| BCM06 | 6450 |
| BCM07 | 7170 |
| BCM08 | 7419 |
| BCM09 | 8149 |
| BCM10 | 8169 |
| BCM11 | 9501 |
| BCM12 | 9700 |
| BCM13 | 9939 |
| BCM 14 | 10050 |
| BCM15 | 11401 |
| BCM16 | 13680 |
| BCM17 | 18730 |

A statistical summary of the experiments is provided in Table 3:

**Table 3**

| **Statistical analysis and classification of the spectra** | | | |
|---|---|---|---|
| **Stage** | **Predictive Value** ^{**[%]**} | **Sensitivity** ^{**[%]**} | **Specificity** ^{**[%]**} |
| Normal | 97 | 72 | 93 |
| DCIS | 78 | 80 | 83 |
| Tumor | 93 | 78 | 84 |

In Table 3 different tumor stages are compared to each other. Diagnostic tests conventionally only compare the healthy with the disease stages. In contrast thereto the present case compares a certain disease stage with the sum of other disease stages. The nomenclature of Table 3 was adopted to reflect this situation.

Detecting early stages of breast cancer with a sensitivity of 78% and a specificity of 84% fully meets the requirements of a suitable diagnostic method.

These examples show that by using differential protein profiling it is possible to diagnose the earliest, non-invasive stages of breast cancer, namely DCIS. Early detection of a respective disease and thus the possibility of an early therapeutic action or a tight screening scheme has the potential to increase cure rates of the patients significantly (up to more than 95%).

Regular surveillance of the protein profiles of serum will further allow to monitor the therapy success of individual patients and will enable better prognosis for the development of and surveillance of the course of the disease. The methods presently used for diagnosis (mammography, ultrasonic analyses, histopathology of biopsies) are significantly improved.

### References

Ardekani, A. M., Liotta, L. A., and Petricoin, E. F., 3rd (2002). Clinical potential ofproteomics in the diagnosis of ovarian cancer. Expert Rev Mol Diagn *2*, 312-320.

Austen, B. M., Frears, E. R., and Davies, H. (2000). The use of seldi proteinchip arrays to monitor production of Alzheimer's betaamyloid in transfected cells. J Pept Sci *6*, 459-469.

Ball, G., Mian, S., Holding, F., Allibone, R. 0., Lowe, J., Ali, S., Li, G., McCardle, S., Ellis, I. 0., Creaser, C., and Rees, R. C. (2002). An integrated approach utilizing artificial neural networks and SELDI mass spectrometry for the classification of human tumours and rapid identification of potential biomarkers. Bioinformatics *18*, 395-404.

Li, J., Zhang, Z., Rosenzweig, J., Wang, Y. Y., and Chan, D. W. (2002). Proteomics and bioinformatics approaches for identification of serum biomarkers to detect breast cancer. Clin Chem *48*, 1296-1304.

Petricoin, E. F., Ardekani, A. M., Hitt, B. A., Levine, P. J., Fusaro, V. A., Steinberg, S. M., Mills, G. B., Simone, C., Fishman, D. A., Kohn, E. C., and Liotta, L. A. (2002). Use of proteomic patterns in serum to identify ovarian cancer. Lancet *359*, 572-577.

Schulze-Garg, C., Lohler, J., Gocht, A., and Deppert, W. (2000). A transgenic mouse model for the ductal carcinoma in situ (DCIS) of the mammary gland. Oncogene *19*, 1028-1037.

Stieber, P., Molina, R., Chan, D. W., Fritsche, H. A., Beyrau, R., Bonfrer, J. M., Filella, X., Comet, T. G., Hoff, T., Jager, W., *et al.* (2001). Evaluation of the analytical and clinical performance of the Elecsys CA 15-3 immunoassay. Clin Chem *47*, 2162-2164.

Petera, J., Filip, S., Slampa, P., Soumarova, R., Coupek, P., and Zatloukal, P. (2001). Management of inoperable carcinoma of the breast by curative radiotherapy and chemo-hormonotherapy. Onkologie *24*, 263-266.

Silverstein, M. J., Barth, A., Poller, D. N., Gierson, E. D., Colbum, W. J., Waisman, J. R., and Gamagami, P. (1995). Ten-year results comparing mastectomy to excision and radiation therapy for ductal carcinoma in situ of the breast. Eur J Cancer *31A*, 1425-1427.

Solin, L. J., Kurtz, J., Fourquet, A., Amalric, R., Recht, A., Bomstein, B. A., Kuske, R., Taylor, M., Barrett, W., Fowble, B., *et al.* (1996). Fifteen-year results of breast-conserving surgery and definitive breast irradiation for the treatment of ductal carcinoma in situ of the breast. J Clin Oncol *14*, 754-763.

Talamonti, M. S. (1996). Management of ductal carcinoma in situ. Semin Surg Oncol *12*, 300-313.

## Claims

1. A method for generating a data base for differential diagnosis of breast cancer stages, which method comprises steps, wherein:
(a) samples are obtained from healthy subjects, from subjects having a ductal carcinoma in situ (DCIS) and from subjects with invasive, ductal breast carcinoma;
(b) protein profiles are analyzed in all samples using mass spectrometry and entered into a data base;
(c) a mathematical algorithm is used to characterize the protein profiles analyzed as specific for healthy subjects, specific for subjects having a ductal carcinoma in situ or specific for subjects having invasive, ductal breast cancer.

2. An in vitro method for differential diagnosis of breast cancer stages, comprising a method for generating a data base according to claim 1 and further comprising steps, wherein
(d) a sample is obtained from a subject;
(e) the protein profile of the sample is analyzed and compared to the data base of (b); and
(f) on the basis of the characteristics of the protein profile the subject is diagnosed as healthy, as having a ductal carcinoma in situ or as having invasive, ductal breast cancer.

3. A method of identifying a protein specific for subjects having ductal carcinoma in situ, comprising a method for generating a data base according to claim 1 and further comprising steps, wherein one or more proteins **characterized** as specific for ductal carcinoma in situ are isolated.

4. A method of identifying a protein specific for subjects having ductal carcinoma in situ according to claim 3, wherein the sequence of the protein isolated is obtained.

5. An in vitro method for differential diagnosis of breast cancer stages, comprising a method of identifying one or more proteins specific for ductal carcinoma in situ according to claim 3 or 4 and further comprising steps, wherein a probe is obtained from a subject at risk of having breast cancer and analyzed for the presence of the protein(s) specific for ductal carcinoma in situ, wherein the subject is diagnosed as having ductal carcinoma in situ if the protein(s) is (are) present in the probe.

6. A method of preparing a protein specific for subjects having ductal carcinoma in situ, comprising a method of identifying a protein according to claim 3 or 4 and recombinant or synthetic preparation of the protein identified.

7. A method according to one of the preceding claims, wherein the subjects used for generating the data base are mice, transgenic mice diagnosed to have a ductal carcinoma in situ, or humans.

8. A method according to one of the preceding claims, wherein the subjects to be diagnosed are humans, specifically women.

9. A method according to one of the preceding claims, wherein the samples obtained in step (a) are blood samples, serum samples and/or biopsy samples.

10. A method according to one of the preceding claims, wherein the samples obtained in step (d) are blood samples or serum samples.

11. A method according to one of the preceding claims, wherein the samples obtained in step (d) are serum samples which are purified by depletion of hemoglobin prior to mass spectrometry.

12. A method according to one of the preceding claims, wherein the mass spectrometry used is surface enhanced laser desorption ionisation mass spectrometry (SELDI-MS).

13. A method according to one of the preceding claims, wherein the proteins of the sample are applied (or coated) onto a surface used for SELDI-MS analysis.

14. A method according to one of the preceding claims, wherein the surface is a weak cation exchange chip or a strong anion exchange chip.

15. A method according to one of the preceding claims, wherein the mass range of 5 to 200 kDa is selected for further analysis.

16. A method according to one of the preceding claims, wherein a training algorithm is used to characterize the protein profiles.

17. A method according to one of the preceding claims, wherein the method of identifying a protein specific for subjects having ductal carcinoma in situ further comprises steps, wherein the samples analyzed via mass spectrometry is applied to a second conventional MALDI-MS-MS for identification of the sequence of the ions.

18. A method according to one of the preceding claims, wherein the method of identifying a protein specific for subjects having ductal carcinoma in situ further comprises 2D-gelelectrophoresis of the proteins of the sample.

19. A method according to claim 18, wherein the protein identified using the SELDI-MS data is identified in the 2D-gelelectrophoresis using the known isoelectric point (pI) and mass.

20. A method according to claim 18 or 19, wherein the protein is subsequently purified from the gel digested to obtain a mixture of peptide fragments.

21. A method according to one of the claims 18 to 20, wherein the fragments are analyzed via MALDI-TOF to obtain will characteristic fingerprints which allows identification of known proteins using sequence data bases.

22. A method according to one of the claims 1 to 16, wherein the method of identifying a protein specific for subjects having ductal carcinoma in situ further comprises steps, wherein the protein of interest is enriched via ion exchange chromatography or size exclusion chromatography and electro spray ionization-MS (ESI-MS) of the enriched protein is used in combination with a second MS to obtain the sequence of the protein of interest.
